(19) Europäisches Patentamt
European Patent Office
Office européen des brevets

(11) **EP 3 300 799 A1**

(12) **EUROPÄISCHE PATENTANMELDUNG**

(43) Veröffentlichungstag:
**04.04.2018 Patentblatt 2018/14**

(21) Anmeldenummer: **16191735.6**

(22) Anmeldetag: **30.09.2016**

(51) Int Cl.:
*B01J 25/02* (2006.01)    *B01J 35/02* (2006.01)
*B01J 35/10* (2006.01)    *B01J 37/08* (2006.01)
*B01J 37/00* (2006.01)    *C07C 29/17* (2006.01)
*C07C 31/20* (2006.01)    *B01J 23/755* (2006.01)
*B01J 35/04* (2006.01)    *B01J 37/02* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Evonik Degussa GmbH**
**45128 Essen (DE)**

(72) Erfinder:
• **BERWEILER, Monika**
**63477 Maintal (DE)**
• **GÖTTLINGER, Markus**
**63517 Rodenbach (DE)**
• **ROOS, Meike**
**63654 Büdingen (DE)**
• **SCHWARZ, Matthias**
**36103 Flieden (DE)**
• **POSS, René**
**63450 Hanau (DE)**

(54) **VERFAHREN UND KATALYSATOR ZUR HERSTELLUNG VON 1,4-BUTANDIOL**

(57) Die vorliegende Erfindung betrifft ein Verfahren zur Herstellung von 1,4-Butandiol (BDO) durch Hydrierung von 2-Butin-1,4-diol (BYD) oder 4-Hydroxybutanal (4-HBA) in Gegenwart eines Katalysators vom Raney-Typ mit einer Schütt-dichte von bis zu 0,8 kg/L.

EP 3 300 799 A1

**Beschreibung**

Die vorliegende Erfindung betrifft ein Verfahren zur Herstellung von 1,4-Butandiol (BDO) und einen Katalysator zur Verwendung in diesem Verfahren.

1,4-Butandiol selbst wird vor allem in der Textil-, Leder-, Lebensmittel- und Pharmaindustrie verwendet. Als Zwischenprodukt wird es hauptsächlich zur Herstellung von thermoplastischen Polyestern benutzt. Außerdem ist BDO eine synthetische Vorstufe in der Herstellung von einigen wichtigen chemischen Intermediaten und Lösungsmittel wie zum Beispiel Tetrahydrofuran (THF), $\gamma$-Butyrolacton oder Pyrrolidin.

Das am häufigsten eingesetzte industrielle Verfahren zur Herstellung von BDO basiert auf einer kontinuierlichen Hydrierung von 2-Butin-1,4-diol (BYD) katalysiert durch modifizierte Nickelkatalysatoren. Eine einstufige Variante dieses Verfahrens wird typischerweise bei 80-160 °C unter einem Druck von circa 300 bar in einem Festbettreaktor durchgeführt. Eine zweistufige Hydrierung von BYD ist auch bekannt, wobei die erste Stufe, in der hauptsächlich 2-Buten-1,4-diol (BED) hergestellt wird, unter einem geringeren Druck von circa 40 bar durchgeführt wird. In der zweiten Stufe wird BED bei 300 bar zum BDO umgesetzt. Auch andere auf Acetylen als Hauptrohstoff basierte Verfahren zur Herstellung von BDO sind bekannt. So kann beispielsweise Allylalkohol durch Hydroformylierung mit Synthesegas (CO + $H_2$) zum 4-Hydroxybutanal (4-HBA) umgesetzt und weiter zum BDO hydriert werden, wobei die beiden Schritte auch gleichzeitig stattfinden können. Weitere Informationen zum BDO sind zum Beispiel in Ullmann's Encyclopedia of Industrial Chemistry, Kapitel "Butanediols, Butenediol, and Butynediol" veröffentlicht online am 15.06.2000, DOI: 10.1002/14356007.a04_455, beschrieben.

Die für die Hydrierung häufig eingesetzten Nickelkatalysatoren vom "Raney-Typ" werden im Allgemeinen hergestellt durch Bildung einer mindestens binären Metalllegierung, meist umfassend Aluminium (Al) und Nickel (Ni), und anschließendes Herauslösen von Aluminium. Beschreibungen solcher Verfahren werden beispielsweise in den Druckschriften US 1628190 A, US 1915473 A, US 2139602, US 2977327 offenbart.

US 2967893 A offenbart den Einsatz einer Aufschlämmung eines pulverförmigen und mit Kupfer als Promotor modifizierten Nickelkatalysators für die Hydrierung von BYD zu BDO.

Die Aktivierung einer Vorstufe des Katalysators kann separat oder in-situ in einem Hydrierungsreaktor zur Herstellung von BDO erfolgen, wie zum Beispiel in EP 0340970 A2 und DE 2004611 A1 beschrieben.

DE 2004611 A offenbart ein kontinuierliches Verfahren zur Herstellung von BDO durch die Hydrierung einer wässrigen Lösung von BYD. Eingesetzt wird ein Festbett körnigen, Öffnungen aufweisenden Nickel-Aluminium-Katalysators, der durch Entfernen von etwa 5 bis 30 % des Aluminiums aus einer im wesentlichen etwa 35 bis 60 Gew.-% Nickel und etwa 40 bis 65 Gew.-% Aluminium bestehenden Nickel-Aluminium-Legierung aktiviert worden ist. Die das Festbett ausbildenden Katalysatorteilchen weisen Korngrößen im Bereich von etwa 2 cm bis 1,4 mm, vorzugsweise von etwa 1 cm bis 2 mm auf.

EP 0807464 A1 offenbart ein Verfahren zur katalytischen Hydrierung von Hydroxyaldehyden und zyklischen Hydroxyethern an einem modifizierten Raney-Nickelkatalysator enthaltend 40-98 Gew.-% Ni, 1-50 Gew.-% Al, 0,05-15 Gew.-% Fe und optional 0,05-10 Gew.-% wenigstens eines Metalls ausgewählt aus der Gruppe bestehend aus Cr, Mo, W, Co, Mn und Ti. Dieser Katalysator kann vom Pulvertyp sein, wenn die Reaktion in einem Schlammbettreaktor durchgeführt wird. Ebenso kann der in EP 0807464 A1 offenbarte Katalysator vom Granulattyp sein, wenn die Reaktion in einem Festbettreaktor durchgeführt wird, wie in DE 2004611 A.

EP 1833778 B1 offenbart ein Verfahren zur Hydrierung von 4-Hydroxybutyraldehyd (HBA) zu 1,4-Butandiol (BDO) und/oder von 2-Methyl-3-hydroxypropionaldehyd (HMPA) zu 2-Methyl-1,3-propandiol, wobei eine wässrige Lösung von HBA und/oder HMPA mit Wasserstoff in einer adiabatischen Hydrierzone kontaktiert wird, durch Kontakt mit einem Festbett eines Hydrierkatalysators, der ein mit Molybdän aktivierter Nickelkatalysator ist.

Der Nachteil der heute bekannten Verfahren zur Herstellung von BDO liegt bei großtechnischer Anwendung im ausgesprochen hohen Bedarf an Nickel, das in diesen Verfahren als Hydrierkatalysator vom Raney-Typ eingesetzt wird. Wie vorstehend beschrieben, werden in kontinuierlichen Verfahren üblicherweise Nickelkatalysatoren vom Granulattyp eingesetzt, die als nicht aktivierte Vorstufe in den Reaktor eingebracht und in situ durch Herauslösen von Aluminium aktiviert werden. Diese Katalysatoren weisen üblicherweise Schüttdichten von mehr als 1,5 kg/L auf, so dass bei Reaktorfüllungen von 5 bis 50 $m^3$ Katalysator, wie sie bei der üblichen Aktivität dieser Katalysatoren zur Erzielung hinreichender Produktausbeuten notwendig sind, zwischen 8 und 100 Tonnen an Nickel zum Einsatz kommen. Nickel gehört mit einem Massenanteil von rund 0,01 % in der Erdhülle zu den eher seltenen Metallen. Die Verfügbarkeit in wirtschaftlich zugänglichen Vorkommen ist begrenzt. Zudem steigt der weltweite Bedarf an Nickel für technische Anwendungen beispielsweise im Elektronik- und Werkstoffsektor stetig an.

Der vorliegenden Erfindung lag daher die Aufgabe zugrunde, ein wirtschaftliches und insbesondere hinsichtlich des Nickelbedarfs möglichst Resourcen schonendes Verfahren zur katalytischen Herstellung von 1,4-Butandiol bereit zu stellen. Eine weitere zugrunde liegende Aufgabe war die Bereitstellung von Katalysatoren, mittels derer die entsprechenden chemischen Reaktionen zu BDO mit hinreichenden Ausbeuten und Selektivitäten unter großtechnisch relevanten Prozeßbedingungen geführt werden können.

Diese Aufgaben werden gelöst durch ein Verfahren zur Herstellung von 1,4-Butandiol durch Hydrierung von 2-Butin-1,4-diol oder 4-Hydroxybutanal, wobei eine wässrige Lösung enthaltend 2-Butin-1,4-diol oder 4-Hydroxybutanal mit Wasserstoff und einem aktivierten Nickelkatalysator, der eine Schüttdichte von nicht mehr als 0,8 kg/L aufweist, in Kontakt gebracht wird.

Das erfindungsgemäße Verfahren findet in Gegenwart von Wasser statt. Bevorzugt werden 1 bis 70 Gew. %-ige wässrige Lösungen von BYD oder 4-HBA als Edukt eingesetzt. Je nach Reaktorkonzept und Verfahrensführung können die mit dem aktivierten Nickelkatalysator in Kontakt gebrachten Eduktlösungen bis zu 60 Gew.-% des Zielproduktes BDO enthalten. Dies gilt insbesondere, wenn die Hydrierreaktoren, in denen das erfindungsgemäße Verfahren durchgeführt wird, Kreislaufreaktoren sind.

Das erfindungsgemäße Verfahren wird bevorzugt bei einem Wasserstoffdruck im Bereich von 10 bis 350 bar durchgeführt. Dabei wird die Hydrierung von 4-HBA bevorzugt bei 10 bis 110 bar, besonders bevorzugt 40 bis 100 bar durchgeführt. Die Hydrierung von BYD zu BDO wird bevorzugt bei einem Wasserstoffdruck im Bereich von 50 bis 350 bar, besonders bevorzugt von 75 bis 320 bar und ganz besonders bevorzugt von 100 bis 300 bar durchgeführt.

Der erfindungsgemäße Prozess kann bei Temperaturen von 50 bis 250 °C durchgeführt werden. Die Hydrierung von 4-HBA zu BDO erfolgt bevorzugt bei 50 bis 200°C, besonders bevorzugt bei 50 bis 150 °C und ganz besonders bevorzugt im Temperaturbereich von 50 bis 100°C. Die Hydrierung von BYD zu BDO wird bevorzugt in einem Temperaturbereich von 50 bis 150°C, besonders bevorzugt bei 70 bis 140°C und ganz besonders bevorzugt im Temperaturbereich zwischen 80 und 135°C durchgeführt.

Das erfindungsgemäße Verfahren zur Hydrierung von BYD oder 4-HBA kann diskontinuierlich oder kontinuierlich durchgeführt werden. Bei diskontinuierlichem Betrieb (Satzbetrieb) in einem Rührkesselreaktor kann der eingesetzte Katalysator lose im Reaktionsgemisch gerührt werden. Nach Beendigung der Umsetzung kann der Katalysator vom Reaktionsgemisch z.B. durch Filtration, Abziehen/Abpumpen der überstehenden Reaktionslösung oder auf eine andere dem Fachmann bekannte Weise abgetrennt werden.

Bevorzugt wird eine Haltevorrichtung für den eingesetzten Katalysator verwendet. Wird das erfindungsgemäße Verfahren diskontinuierlich in einem Rührkesselreaktor durchgeführt, so ist der Katalysator bevorzugt in einer Haltevorrichtung nahe der Rührwelle so angeordnet, dass durch den Rührer eine Strömung des Reaktionsgemisches durch die in die Haltevorrichtung eingebrachte Katalysatorschüttung erzeugt wird. Diese Ausführungsform hat gegenüber dem losen, d.h. nicht fixierten Einsatz des Katalysators den Vorteil, dass eine nachfolgende Abtrennung des Produktgemisches vom Katalysator in einem zusätzlichen Verfahrensschritt nicht benötigt wird.

Bevorzugt wird das erfindungsgemäße Verfahren kontinuierlich in einem Festbettreaktor durchgeführt, beispielsweise in einem Rieselbett- oder Sumpfreaktor, in einer Blasensäule oder in einem anderen, der Fachwelt bekannten Reaktortypen. Alle diese Reaktortypen können in einem "once through" Modus betrieben werden, bei dem die Reaktanten (Feed) in den Reaktor eingeleitet werden und das Produktgemisch nach der Reaktion abgeführt wird. Alternativ kann ein Teil des Produktgemisches aus dem Reaktor zurück in die Reaktionszone geleitet werden (Kreislaufstrom). Bei einer solchen Kreislaufführung (Recyclingbetrieb) beträgt das Gewichtsverhältnis von Feed zu Kreislaufstrom 0,025 bis 0,25, bevorzugt 0,05 bis 0,15, besonders bevorzugt 0,05 bis 0,1.

Die Umsetzung von BYD zu BDO kann einstufig oder zweistufig durchgeführt werden.

Die einstufige Reaktionsführung der Hydrierung von BYD zu BDO erfolgt bevorzugt kontinuierlich, wobei der Katalysator als Festbettschüttung in einem adiabatisch betriebenen Reaktor vorliegt. Die Temperatur im Reaktoreintritt liegt dann bevorzugt im Bereich von 80 bis 100°C, die Temperatur im Reaktoraustritt zwischen 110-150°C. Dabei ergibt sich bevorzugt eine Temperatur in der Reaktionszone, in der die Hydrierreaktion zum BDO abläuft, die in einem Bereich zwischen 110 und 135°C liegt.

Bei Ausführung der erfindungsgemäßen Hydrierung von BYD zu BDO im diskontinuierlich betriebenen Rührkesselreaktor wird bevorzugt eine zweistufige Temperaturführung gewählt. Hierzu wird die Temperatur im Rührkessel zu Beginn der Reaktion im Bereich von 90 bis 105°C gehalten, so dass Butin-1,4-diol mit Wasserstoff mindestens teilweise zu Buten-1,4-diol umgesetzt wird. Eine ideale Haltezeit bis zur möglichst vollständigen Umsetzung von BYD zu Buten-1,4-diol kann durch Detektion der während dieser Zeit aufgenommenen Menge an Wasserstoff bestimmt werden. Nach Aufnahme des stöchiometrischen Wasserstoffäquivalents zur eingesetzten Menge BYD ist die erste Reaktionsstufe abgeschlossen. Dann wird die Temperatur im Rührkessel auf 130 bis 135°C erhöht und bis zur Vervollständigung der Hydrierung zu 1,4-Butandiol gehalten.

Für eine optimale Reaktionsführung des erfindungsgemäßen Verfahrens wird die Hydrierung bei pH-Werten zwischen 4,0 bis 9,0 durchgeführt.

Aktivierte Nickelkatalysatoren sind dem Fachmann auch als Katalysatoren vom "Raney-Typ", oder einfach "Raney-Nickelkatalysatoren" grundsätzlich bekannt. Sie werden im Allgemeinen hergestellt durch Bildung einer mindestens binären Metalllegierung, meist umfassend Aluminium (Al) und Nickel (Ni), und anschließendes Herauslösen von Aluminium.

Die im erfindungsgemäßen Verfahren eingesetzten aktivierten Nickelkatalysatoren weisen eine Schüttdichte von nicht mehr als 0,8 kg/L, bevorzugt von 0,1 bis 0,7 kg/L, besonders bevorzugt von 0,2 bis 0,6 kg/L auf.

Schüttdichte $d_{Sch}$, auch manchmal Fülldichte (Engl. bulk density, poured density) eines Feststoffes genannt, ist das Verhältnis der Masse zum Volumen eines Gemenges aus einem körnigen Feststoff und Luft, welche die Hohlräume zwischen den Partikeln ausfüllt. Dieser unter Fachleuten gängige Parameter kann mittels eines Messzylinders, durch Bestimmung der Masse ($M_F$) eines definierten Schüttungsvolumens an Feststoff ($V_F$) bestimmt werden:

$$d_{Sch} = M_F/V_F$$

Die im erfindungsgemäßen Verfahren eingesetzten aktivierten Nickelkatalysatoren enthalten 65 bis 98 Gew.-%, bevorzugt 70 bis 95 Gew.-%, besonders bevorzugt 80 bis 90 Gew. % Nickel und 0 bis 15 Gew.-%, bevorzugt 0 bis 13 Gew.-%, besonders bevorzugt 4 bis 13 Gew.-%, ganz besonders bevorzugt 7 bis 13 Gew. % Aluminium. Weiterhin werden in bevorzugten Ausführungsformen des erfindungsgemäßen Verfahrens aktivierte Nickelkatalysatoren mit bis zu 10 Gew. %, bevorzugt 0,05 bis 5 Gew. %, besonders bevorzugt 0,1 bis 2 Gew. % Molybdän (Mo) und/oder 0 bis 10 Gew.-%, bevorzugt 0,05 bis 5 Gew.-%, besonders bevorzugt 1,5 bis 3,5 Gew.-% eines oder mehrerer Elemente ausgewählt aus der Gruppe bestehend aus Eisen und Chrom eingesetzt.

Die im erfindungsgemäßen Verfahren eingesetzten aktivierten Nickelkatalysatoren weisen bevorzugt eine durchschnittliche Teilchengröße von maximal 70 mm auf. Grundsätzlich ist die optimale Teilchengröße steuerbar und wird auf die im Einsatzreaktor herrschenden Gegebenheiten abgestimmt. Bevorzugt werden die aktivierten Nickelkatalysatoren als Schüttgut eingesetzt mit einer Teilchengröße von bis zu 50 mm, besonders bevorzugt mit einer Teilchengröße von bis zu 30 mm und ganz besonders bevorzugt von nicht mehr als 10 mm.

Die durchschnittliche Teilchengröße für die Teilchen mit einer Größe im Bereich von 5 $\mu$m bis 125 mm kann durch Siebanalyse nach DIN 66165 bestimmt werden. Alternativ kann eine durchschnittliche Teilchengröße mittels eines Mikroskops optisch erfasst werden, wobei ein Zahlenmittelwert aus mindestens 100 einzelnen Werten zu bestimmen ist.

Die im erfindungsgemäßen Verfahren eingesetzten aktivierten Nickelkatalysatoren weisen eine BET Oberfläche von 1 bis 200 m$^2$/g, bevorzugt 10 bis 120 m$^2$/g, besonders bevorzugt 70 bis 100 m$^2$/g auf. Die spezifische Oberfläche, auch vereinfacht BET Oberfläche genannt, wird nach DIN 9277 durch Stickstoffadsorption nach dem Brunauer-Emmett-Teller-Verfahren bestimmt, wie beschrieben in J. Am. Chem. Soc. 1938, Vol. 60, S. 309-319.

Die im erfindungsgemäßen Verfahren eingesetzten aktivierten Nickelkatalysatoren weisen bevorzugt eine makroskopische Schaumstruktur auf. Poröse Metallschaumstrukturen, die viele Hohlräume enthalten, können zum Beispiel durch Einwirkung von Gasen auf ein verflüssigtes Metall und nachfolgende Abkühlung entstehen. Eine weitere Möglichkeit, um zu solchen Strukturen zu gelangen, besteht darin, organische Schaumstrukturen als Templat (Basis) für die Auftragung eines Metalls zu benutzen und anschließend das organische Templat durch Verbrennung zu entfernen.

Die im erfindungsgemäßen Verfahren eingesetzten aktivierten Nickelkatalysatoren zeigen bevorzugt eine poröse Schaumstruktur, wobei die makroskopischen Poren Größen im Bereich von 100 bis 5000 $\mu$m, bevorzugt von 200 bis 2500 $\mu$m, besonders bevorzugt von 400 bis 1200 $\mu$m aufweisen. Zur Bestimmung der Größe der makroskopischen Poren kann zum Beispiel eine in "The Guide 2000 of Technical Foams", Buch 4, Teil 4, Seiten 33-41 beschriebene Methode benutzt werden. Dabei kann die Größe der makroskopischen Poren durch eine optische Messung des Porendurchmessers einer ausgewählten Pore bestimmt werden. Diese Messung wird für mindestens 100 unterschiedliche Poren wiederholt, ein Mittelwert des Porendurchmessers daraus dann als Analyseergebnis berechnet.

Ein weiterer Gegenstand der Erfindung ist ein aktivierter Nickelkatalysator, der eine Schüttdichte von nicht mehr als 0,8 kg/L aufweist. Insbesondere kann dieser aktivierte Nickelkatalysator in einem Verfahren zur Herstellung von 1,4-Butandiol durch Hydrierung von 2-Butin-1,4-diol oder 4-Hydroxybutanal, wobei eine wässrige Lösung enthaltend 2-Butin-1,4-diol oder 4-Hydroxybutanal mit Wasserstoff und einem aktivierten Nickelkatalysator in Kontakt gebracht werden, eingesetzt werden.

Alle vorstehend aufgeführten, auf die im erfindungsgemäßen Verfahren eingesetzten Katalysatoren bezogenen Definitionen und Erläuterungen bezüglich der Analysenmethoden gelten entsprechend für die nachstehend beschriebenen erfindungsgemäßen aktivierten Nickelkatalysatoren.

Die erfindungsgemäßen aktivierten Nickelkatalysatoren weisen eine Schüttdichte von nicht mehr als 0,8 kg/L, bevorzugt von 0,1 bis 0,7 kg/L, besonders bevorzugt von 0,2 bis 0,6 kg/L auf.

Die erfindungsgemäßen aktivierten Nickelkatalysatoren enthalten 65 bis 98 Gew.-%, bevorzugt 70 bis 95 Gew.-%, besonders bevorzugt 80 bis 90 Gew. % Nickel und 0 bis 15 Gew.-%, bevorzugt 0 bis 13 Gew.-%, besonders bevorzugt 4 bis 13 Gew.-%, ganz besonders bevorzugt 7 bis 13 Gew. % Aluminium. Weiterhin enthalten bevorzugte Ausführungsformen des erfindungsgemäßen aktivierten Nickelkatalysators bis zu 10 Gew. %, bevorzugt 0,05 bis 5 Gew. %, besonders bevorzugt 0,1 bis 2 Gew. % Molybdän (Mo) und/oder 0 bis 10 Gew.-%, bevorzugt 0,05 bis 5 Gew.-%, besonders bevorzugt 1,5 bis 3,5 Gew.-% eines oder mehrerer Elemente ausgewählt aus der Gruppe bestehend aus Eisen und Chrom.

Die erfindungsgemäßen aktivierten Nickelkatalysatoren weisen bevorzugt eine durchschnittliche Teilchengröße von maximal 70 mm auf. Grundsätzlich ist die optimale Teilchengröße steuerbar und wird auf die im Einsatzreaktor herrschenden Gegebenheiten abgestimmt. Bevorzugt werden die erfindungsgemäßen aktivierten Nickelkatalysatoren als Schüttgut eingesetzt mit einer Teilchengröße von bis zu 50 mm, besonders bevorzugt mit einer Teilchengröße von bis zu 30 mm und ganz besonders bevorzugt von nicht mehr als 10 mm.

Die erfindungsgemäßen aktivierten Nickelkatalysatoren weisen eine BET Oberfläche von 1 bis 200 m$^2$/g, bevorzugt 10 bis 120 m$^2$/g, besonders bevorzugt 70 bis 100 m$^2$/g auf.

Die erfindungsgemäßen aktivierten Nickelkatalysatoren weisen bevorzugt eine makroskopische Schaumstruktur auf, wobei die makroskopischen Poren Größen im Bereich von 100 bis 5000 $\mu$m, bevorzugt von 200 bis 2500 $\mu$m, besonders bevorzugt von 400 bis 1200 $\mu$m aufweisen.

Zur Herstellung eines erfindungsgemäßen aktivierten Nickelkatalysators wird ein Nickelmetallschaum mit einem Haftvermittler besprüht, mit Aluminiumpulver beschichtet und das so erhaltene Material einer Wärmebehandlung unterzogen. Dann erfolgt eine Verkleinerung, Vereinzelung und/oder Formgebung des nach der Wärmebehandlung erhaltenen Ni/Al-Materials. Der erfindungsgemäße aktivierte Nickelkatalysator wird daraus durch Herauslösen mindestens eines Teils des darin enthaltenen Aluminiums erhalten.

Der zur Herstellung des erfindungsgemäßen Katalysators zu verwendende Nickelmetallschaum wird bevorzugt in Plattenform mit Kantenlängen bis zu 500 mm und einer Dicke von nicht mehr als 5 mm eingesetzt. Um die Haftung von Aluminiumpulver auf dem Nickelschaum zu verbessern, wird dieser zunächst mit einem Haftvermittler behandelt. Jeder Haftvermittler, der die Haftung zwischen Metallen und organischen Materialen verbessert, kann eingesetzt werden. Geeignet ist beispielsweise Polyethyleniminlösung.

Nach dem Auftragen des Aluminiumpulvers auf den Nickelmetallschaum wird das Material einer Wärmebehandlung im Temperaturbereich von 500 bis 1000 °C, bevorzugt von 600 bis 800°C unterzogen, wobei zunächst Feuchtigkeit und organische Reststoffe aus dem vorhergehenden Beschichtungsprozess entfernt und anschließend Aluminium mindestens teilweise verflüssigt und in die Nickelschaumstruktur einlegiert wird.

Die Wärmebehandlung erfolgt in einer Atmosphäre aus Sauerstoff-freiem Inertgas, um die Ausbildung störender oxidischer Schichten zu verhindern.

Das so erhaltene Material, ein mit Aluminium modifizierter Nickelmetallschaum, wird dann gegebenenfalls zerkleinert, vereinzelt und/oder einer entsprechenden Formgebung unterzogen. Die Zerkleinerung und Vereinzelung des mit Aluminium modifizierten Nickelmetallschaums kann beispielsweise durch Laserschneiden bzw. Laserstrahlschneiden erfolgen. Die entstehenden Materialstücke (Teilchen, Partikel) weisen bevorzugt eine Quader- oder Parallelepipedform mit einer maximalen Kantenlänge von nicht mehr als 50 mm auf und bilden in ihrer Gesamtheit ein Schüttgut.

Im nächsten Schritt wird der erfindungsgemäße Katalysator durch Aktivierung des mit Aluminium modifizierten Nickelschaums gewonnen. Hierzu wird mindestens ein Teil des Aluminiums chemisch aus dem Material herausgelöst. Dazu werden wässrige basische Lösungen eingesetzt, bevorzugt Alkalihydroxidlösungen ausgewählt aus der Gruppe bestehend aus Natriumhydroxid, Kaliumhydroxid oder Lithiumhydroxid, besonders bevorzugt wässrige Natriumhydroxidlösung. Die Konzentration der bei der Katalysatorherstellung eingesetzten wässrigen Alkalihydroxidlösung kann generell zwischen 0,1 und 60 Gew. -% sein. Bevorzugt erfolgt das Herauslösen des Aluminiums (auch als Laugung bezeichnet) mit einer 5 bis 50 Gew.-%igen, besonders bevorzugt 5 bis 35 Gew.- %igen wässrigen Natriumhydroxidlösung bei einer Temperatur von 20 bis 100°C, bevorzugt bei 40 bis 85°C, besonders bevorzugt bei 50 bis 80°C. Die dabei anzuwendenden Laugzeiten, d.h. die Reaktionszeiten der Natriumhydroxidlösung mit dem mit Aluminium modifizierten Nickelmetallschaum, hängen außer von den sonstigen, vorstehend benannten Reaktionsbedingungen vom einzustellenden Aluminiumgehalt im Endprodukt ab und können zwischen 2 und 240 Minuten liegen.

Im im Ergebnis erzeugten erfindungsgemäßen aktivierten Nickelkatalysator bleibt die makroskopische Schaumstruktur des ursprünglich eingesetzten Nickelschaums erhalten. Das mindestens teilweise Herauslösen des Aluminiums erfolgt in Oberflächen-nahen Bereichen, wo eine hochporöse, katalytisch aktive Nickelstruktur erzeugt wird. Die BET Oberfläche des erfindungsgemäßen Katalysators ist bevorzugt größer als die des eingesetzten Nickelmetallschaums.

Zur Verbesserung von Aktivität, Selektivität und/oder Standzeit des erfindungsgemäßen aktivierten Nickelkatalysators können unterschiedliche Dotierungen und/oder Promotoren zugesetzt werden. Dies kann durch Einlegierung eines Dotierungsmetalls in den zur Katalysatorherstellung eingesetzten Nickelmetallschaum und/oder durch naßchemische Nachbehandlung des erfindungsgemäßen aktivierten Nickelkatalysators erfolgen. Beispielsweise kann das Dotierungsmetall durch Fällung oder reduktive Zersetzung aus bevorzugt wässriger Lösung einer geeigneten Vorstufe aufgebracht werden. Bevorzugt werden ein oder mehrere Dotierungselemente aufgebracht, ausgewählt aus der Gruppe bestehend aus Platin (Pt), Palladium (Pd), Rhodium (Rh), Ruthenium (Ru), Osmium (Os), Iridium (Ir), Eisen (Fe), Cobalt (Co), Chrom (Cr), Molybdän (Mo), Wolfram (Wo), Mangan (Mn), Rhenium (Re), Kupfer (Cu), Silber (Ag) und Gold (Au) eingesetzt werden. Besonders bevorzugt sind Fe, Co, Cr und/oder Mo.

Die Erfindung wird im Folgenden anhand einiger Beispiele und Figuren näher erläutert.

**Beispiel 1:**

Ein kommerziell als Rollenware erhältlicher Nickelschaum mit einer Dicke von 1,9 mm, einer Breite von 300 mm und einer mittleren Porengröße von 580 μm wurde mit einer kommerziell erhältlichen Haftvermittlerlösung besprüht, mit Aluminiumpulver beschichtet und einer Wärmebehandlung bei 700°C unterzogen. Nach Abkühlung wurde das so erhaltene Material mit einem Laser in viereckige Teilchen mit einer Kantenlänge von 4 mm x 4 mm und einer Dicke von 1,9 mm geschnitten.

Das resultierende Schüttgut wurde zur katalytischen Aktivierung in einer Festbettschüttung angeordnet und durch Durchpumpen von 5M NaOH-Lösung (Natronlauge) naßchemisch nachbehandelt. Eine Teilmenge A des Schüttguts wurde dieser naßchemischen Nachbehandlung bei 70°C für die Dauer von 5 Minuten unterzogen. Eine Teilmenge B des Schüttguts wurde bei 60°C für die Dauer von 15 Minuten mit Natronlauge nachbehandelt.

Beide Teilmengen wurden anschließend mit Wasser solange gewaschen, bis ein pH-Wert der Waschlösung nach Durchpumpen durch die Festbettschüttung < 10 erreicht war.

Die Zusammensetzung der beiden so erhaltenen, katalytisch aktiven Schüttgutteilmengen wurde per ICP-OES analysiert. Die Ergebnisse sind in der nachfolgenden Tabelle angegeben:

| Katalysator | Nickelgehalt | Aluminiumgehalt | Schüttdichte |
|---|---|---|---|
| A | 85,8 Gew.-% | 14,1 Gew.-% | 0,5 kg/L |
| B | 85,3 Gew.-% | 14,7 Gew.-% | 0,5 kg/L |

Der Katalysator A wurde in einen Rührkesselreaktor mit einem Gesamtvolumen von 500 ml eingebracht, um die katalytischen Wirksamkeit in der Hydrierung von Butin-1,4-diol (BYD) zu 1,4-Butandiol (BDO) zu untersuchen. In dem Reaktor wurden 300 mL Wasser vorgelegt, 5 mL des Katalysators A wurden in ein nahe der Rührwelle unterhalb des Wasserspiegels positioniertes Körbchen eingebracht. Nach Schließen des Reaktors, Atmosphärenaustausch und Befüllen des Reaktors mit Wasserstoff bis zu einem Druck von 80 bar wurden 86,6 g BYD in 50%-iger wässriger Lösung in den Reaktor unter Rühren eingepumpt und der Reaktor wurde auf 100°C aufgeheizt. Nach einer Reaktionszeit von 100 Minuten wurde die Temperatur im Reaktor auf 135°C erhöht und weitere 260 Minuten gehalten. Nach Abkühlung auf Raumtemperatur wurde eine Probe des Reaktionsgemisches entnommen und mit Gaschromatographie analysiert. Aus den gemessenen Konzentrationen der Bestandteile des Reaktionsgemisches wurden der Butindiol-Umsatz, die Ausbeute zu BDO und die Selektivität zu BDO, sowie die Raumzeitausbeute $RZA_{BDO,V}$ bezogen auf das Katalysatorvolumen bestimmt. Die Ergebnisse sind in der nachstehenden Tabelle angegeben:

| Umsatz BYD | Ausbeute BDO | Selektivität zu BDO | $RZA_{BDO,V}$ |
|---|---|---|---|
| 97 % | 86 % | 89 % | 1,30 kg/(L Kat*h) |

Die angegebenen Größen berechnen sich wie folgt:

- BYD-Umsatz ist definiert als die Stoffmenge des verbrauchten BYD bezogen auf die eingesetzte Stoffmenge BYD:

$$U_{BYD}[\%] = \frac{n_0(BYD) - n(BYD)}{n_0(BYD)} * 100,$$

mit no (BYD) = eingesetzte Stoffmenge BYD und
n (BYD) = Stoffmenge BYD zum Reaktionsende

- Ausbeute BDO ist definiert als die erhaltene Stoffmenge BDO bezogen auf die eingesetzte Stoffmenge BYD:

$$A_{BDO}[\%] = \frac{n(BDO)}{n_0(BYD)} * 100$$

- Selektivität zu BDO ist definiert als das Verhältnis aus der gebildeten Menge des Wunschproduktes BDO zur umgesetzten Menge des Edukts BYD:

$$S_{BDO}[\%] = \frac{n(BDO)}{n_0(BYD) - n(BYD)} * 100$$

- Die Katalysatorvolumen-bezogene Raumzeitausbeute ist definiert als Produktionsleistung bezogen auf das Volumen des Katalysators (in Liter), wobei unter Produktionsleistung die pro Reaktionslauf erzeugte Masse des Wunschproduktes BDO (in kg) bezogen auf die Reaktionszeit t (in Stunden) verstanden wird:

$$RZA_{BDO,V} = \frac{m(BDO)}{V_{Kat} * t}$$

**Beispiel 2:**

Es wurde ein Katalysator hergestellt wie in Beispiel 1 beschrieben, wobei die naßchemische Nachbehandlung mit 10-Gew.-%iger Natronlauge bei 60°C für die Dauer von 60 Minuten durchgeführt wurde. Die Analyse per ICP-OES ergab eine Zusammensetzung des resultierenden katalytisch aktiven Schüttguts (Katalysator C) aus 89 Gew.-% Nickel und 11 Gew.-% Aluminium. Das Material wies eine Schüttdichte von $d_{Sch}$ = 0,3 kg/L auf.
Auch Katalysator C wurde in einem Rührkesselreaktor auf seine katalytische Wirksamkeit in der Hydrierung von Butin-1,4-diol (BYD) zu 1,4-Butandiol (BDO) untersucht. Versuchsaufbau, - durchführung und -auswertung erfolgten wie in Beispiel 1 beschrieben.
Die Ergebnisse sind in der nachstehenden Tabelle angegeben:

| Umsatz BYD | Ausbeute BDO | Selektivität zu BDO | $RZA_{BDO,V}$ |
|------------|--------------|---------------------|---------------|
| 97 % | 90 % | 93 % | 1,37 kg/(L Kat*h) |

Aus der Schüttdichte des Katalysators C und der Katalysatorvolumen-bezogenen Raumzeitausbeute ließ sich die auf die Katalysatormasse bezogene Raumzeitausbeute $RZA_{BDO,m}$ wie folgt berechnen:

$$RZA_{BDO,m} = RZA_{BDO,V} / d_{Sch}$$

Sie betrug $RZA_{BDO,m}$ = 4,55 kg/(kg Kat*h).

**Beispiel 3:**

Es wurde ein Katalysator hergestellt, wie in Beispiel 1 beschrieben, wobei die naßchemische Nachbehandlung mit 10-Gew.-%iger Natronlauge bei 80°C für die Dauer von 90 Minuten durchgeführt wurde. Nach Abschluß der naßchemischen Behandlung mit Natronlauge wurde auf dem Katalysator Molybdän aus einer wässrigen Molybdatlösung abgeschieden. Die Analyse per ICP-OES ergab eine Zusammensetzung des resultierenden katalytisch aktiven Schüttguts (Katalysator D) aus 91 Gew.-% Nickel, 8,7 Gew.-% Aluminium und 0.3 Gew.-% Molybdän. Das Material wies eine Schüttdichte von $d_{Sch}$ = 0,32 kg/L auf.
Der Katalysator D wurde in einem Pilotfestbettreaktor zur Hydrierung von 4-Hydroxybutanal (HBA) zu BDO bei einer Temperatur von 60°C und einem Wasserstoffdruck von 100 bar eingesetzt und zeigte nahezu quantitativen HBA-Umsatz bei sehr guten BDO-Ausbeuten und Selektivitäten zu BDO.

**Vergleichsbeispiel:**

Es wurde ein aktivierter Nickelkatalysator vom Granulattyp hergestellt, wie er aus dem Stand der Technik, z.B. DE 2004611 A, bekannt ist und in großtechnisch üblichen Anlagen zur Herstellung von BDO eingesetzt wird. Hierzu wurde durch Aufschmelzen von Nickel und Aluminium eine Legierung bestehend aus 42 Gew.-% Nickel und 58 Gew.-% Aluminium hergestellt, mechanisch zerkleinert und ausgesiebt, so dass sich eine Kornfraktion von 1,8 bis 4 mm Korngröße ergab. Diese Legierungsgranulatfraktion wurde in einer Festbettschüttung durch Durchpumpen von 10 Gew.-%iger Natronlauge bei 60°C für die Dauer von 60 Minuten katalytisch aktiviert und anschließend mit Wasser gewaschen, bis in der resultierenden Waschlösung ein pH-Wert < 10 erreicht war. Der resultierende Katalysator wies eine Schüttdichte von $d_{Sch}$ = 1,7 kg/L auf. Der Aluminiumgehalt lag bei etwa 37 Gew.-% Aluminium.
Auch dieser Katalysator nach dem Stand der Technik wurde in einem Rührkesselreaktor auf seine katalytische Wirksamkeit in der Hydrierung von Butin-1,4-diol (BYD) zu 1,4-Butandiol (BDO) untersucht. Versuchsaufbau, -durchführung und -auswertung erfolgten wie in Beispiel 1 beschrieben.
Die Ergebnisse sind in der nachstehenden Tabelle angegeben:

| Umsatz BYD | Ausbeute BDO | Selektivität zu BDO | RZA$_{BDO,V}$ |
|---|---|---|---|
| 97 % | 85 % | 88 % | 1,29 kg/(L Kat*h) |

Aus der Schüttdichte des Katalysators nach dem Stand der Technik und der Katalysatorvolumen-bezogenen Raumzeitausbeute ließ sich die auf die Katalysatormasse bezogene Raumzeitausbeute RZA$_{BDO,m}$ berechnen. Sie betrug 0,75 kg/(kg Kat*h).

Die in den Rührkesselversuchen erhaltenen Ergebnisse für die Katalysatoren A (aus Beispiel 1) und C (aus Beispiel 2) werden verglichen mit den mit dem Katalysator nach dem Stand der Technik erhaltenen Kenndaten (Vergleichsbeispiel): bei gleichbleibendem Umsatz zeigt Katalysator A eine leichte Verbesserung, Katalysator C zeigt eine erhebliche Verbesserung von BDO-Ausbeute und Selektivität zu BDO.

Weiterhin werden die auf die Katalysatormasse bezogenen Raumzeitausbeuten, die mit dem Katalysator vom Granulattyp nach dem Stand der Technik (Vergleichsbeispiel) und dem erfindungsgemäßen Katalysator C erzielt wurden, verglichen. Deutlich zu erkennen ist eine Versechsfachung des Wertes für den erfindungsgemäßen Katalysator C im Vergleich zum Stand der Technik.

Die sich daraus ergebende Mengeneinsparung an Nickel für einen großtechnischen Festbettreaktor wird im Folgenden am Beispiel eines typischen, 20 m$^3$ Katalysatorbett umfassenden BDO-Reaktors berechnet. Bei vollkontinuierlicher Fahrweise wird eine solche Anlage im Schnitt mindestens 8000 Betriebsstunden pro Jahr produktiv betrieben. Bei einer Katalysatorvolumen-bezogenen Raumzeitausbeute von 1,29 kg/(L Kat*h) = 1,29 to/(m$^3$ Kat*h) für den Nickelkatalysator vom Granulattyp nach dem Stand der Technik (Vergleichsbeispiel) ergibt sich daraus eine jährlich produzierte BDO-Menge von 1,29 x 20 x 8000 to = 206400 to BDO. Mit dem Katalysator nach dem Stand der Technik, der eine Schüttdichte von 1,7 kg/L = 1,7 to/m$^3$ bei einem Nickelgehalt von 60 - 65 Gew.-% aufweist, werden dafür mindestens 20 x 1,7 x 0,6 to = 20,4 to Nickel benötigt.

Um die gleiche Menge an BDO am Katalysator C aus Beispiel 2, der eine Katalysatorvolumen-bezogene Raumzeitausbeite von 1,37 kg/(L Kat*h) = 1,37 to/(m$^3$ Kat*h) aufweist, zu produzieren, werden 206400 /(1,37*8000) m$^3$ = 18,83 m$^3$ Katalysator benötigt. Das entspricht einer Katalysatorvolumeneinsparung von 1,17 m$^3$ Katalysator oder 5,85 %. Die benötigte Menge Katalysator C liegt bei einer Schüttdichte des erfindungsgemäßen Katalysators C von 0,3 kg/L = 0,3 to/m$^3$ bei 18,83 x 0,3 to = 5,649 to Katalysator C. Das entspricht bei einem Nickelgehalt von 89 Gew.-% einem Nickelbedarf von 5 to gegenüber einem Nickelbedarf von mindestens 20,4 to mit Katalysator vom Granulattyp nach dem Stand der Technik.

Somit kann durch das erfindungsgemäße Verfahren die benötigte Menge an Nickel auf ¼ der heute üblichen Menge reduziert werden, womit ein äußerst effizientes Verfahren zur Herstellung von 1,4-Butandiol bereitgestellt wird.

## Patentansprüche

1.  Verfahren zur Herstellung von 1,4-Butandiol durch Hydrierung von 2-Butin-1,4-diol oder 4-Hydroxybutanal,
    **dadurch gekennzeichnet, dass**
    eine wässrige Lösung enthaltend 2-Butin-1,4-diol oder 4-Hydroxybutanal mit Wasserstoff und einem aktivierten Nickelkatalysator, der eine Schüttdichte von nicht mehr als 0,8 kg/L aufweist, in Kontakt gebracht wird.

2.  Verfahren nach Anspruch 1,
    **dadurch gekennzeichnet, dass**
    die Hydrierung von 4-Hydroxybutanal bei einem Wasserstoffdruck im Bereich von 10 bis 110 bar durchgeführt wird.

3.  Verfahren nach Anspruch 2,
    **dadurch gekennzeichnet, dass**
    die Hydrierung in einem Temperaturbereich von 50 bis 200°C durchgeführt wird.

4.  Verfahren nach Anspruch 1,
    **dadurch gekennzeichnet, dass**
    die Hydrierung von Butin-1,4-diol bei einem Wasserstoffdruck im Bereich von 50 bis 350 bar durchgeführt wird.

5.  Verfahren nach Anspruch 4,
    **dadurch gekennzeichnet, dass**
    die Hydrierung in einem Temperaturbereich von 50 bis 150°C durchgeführt wird.

6. Verfahren nach Anspruch 4 oder 5,
**dadurch gekennzeichnet, dass**
die Hydrierung kontinuierlich durchgeführt wird, wobei der Katalysator als Festbettschüttung in einem adiabatisch betriebenen Reaktor vorliegt, die Temperatur im Reaktoreintritt im Bereich von 80 bis 100°C und die Temperatur im Reaktoraustritt im Bereich von 110 bis 150°C liegt.

7. Verfahren nach einem der Ansprüche 4 oder 5,
**dadurch gekennzeichnet, dass**
die Hydrierung diskontinuierlich in einem Rührkesselreaktor durchgeführt wird, wobei der Katalysator in einer Haltevorrichtung nahe der Rührwelle so angeordnet ist, dass durch den Rührer eine Strömung des Reaktionsgemisches durch die Katalysatorschüttung erzeugt wird.

8. Verfahren nach Anspruch 7,
**dadurch gekennzeichnet, dass**
die Temperatur im Rührkessel zu Beginn der Reaktion im Bereich von 90 bis 105°C gehalten wird, so dass Butin-1,4-diol mit Wasserstoff mindestens teilweise zu Buten-1,4-diol umgesetzt wird, und dann auf 130 bis 135°C erhöht und bis zur Vervollständigung der Hydrierung zu 1,4-Butandiol gehalten wird.

9. Verfahren nach einem der Ansprüche 1 bis 8,
**dadurch gekennzeichnet, dass**
der aktivierte Nickelkatalysator einen Aluminiumgehalt von nicht mehr als 15 Gew. -% aufweist.

10. Verfahren nach einem der Ansprüche 1 bis 9,
**dadurch gekennzeichnet, dass**
der aktivierte Nickelkatalysator eine poröse Schaumstruktur aufweist, wobei die makroskopischen Poren Größen im Bereich von 100 bis 5000 $\mu$m aufweisen.

11. Verfahren nach einem der Ansprüche 1 bis 10,
**dadurch gekennzeichnet, dass**
der aktivierte Nickelkatalysator 0 bis 10 Gew.-% mindestens eines der Elemente ausgewählt aus der Gruppe bestehend aus Molybdän, Eisen und Chrom enthält.

12. Aktivierter Nickelkatalysator, der eine Schüttdichte von nicht mehr als 0,8 kg/L aufweist.

13. Aktivierter Nickelkatalysator nach Anspruch 12,
**dadurch gekennzeichnet, dass**
der Katalysator eine mittlere Teilchengröße von maximal 70 mm aufweist.

14. Aktivierter Nickelkatalysator nach Anspruch 12 oder 13,
**dadurch gekennzeichnet, dass**
der Katalysator als Schüttgut aus quaderförmigen Teilchen mit Kantenlängen von nicht mehr als 50 mm vorliegt.

15. Aktivierter Nickelkatalysator nach einem der Ansprüche 12 bis 14,
**dadurch gekennzeichnet, dass**
der Katalysator einen Aluminiumgehalt von nicht mehr als 15 Gew. -% aufweist.

16. Aktivierter Nickelkatalysator nach einem der Ansprüche 12 bis 15,
**dadurch gekennzeichnet, dass**
der Katalysator eine poröse Schaumstruktur aufweist, wobei die makroskopischen Poren Größen im Bereich von 100 bis 5000 $\mu$m aufweisen.

17. Aktivierter Nickelkatalysator nach einem der Ansprüche 12 bis 16,
**dadurch gekennzeichnet, dass**
der Katalysator 0,05 bis 10 Gew.-% mindestens eines der Elemente ausgewählt aus der Gruppe bestehend aus Molybdän, Eisen und Chrom enthält.

Europäisches
Patentamt

European
Patent Office

Office européen
des brevets

**EUROPÄISCHER RECHERCHENBERICHT**

Nummer der Anmeldung

EP 16 19 1735

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 02/055453 A2 (DEGUSSA [DE]) 18. Juli 2002 (2002-07-18) | 1,4-17 | INV. B01J25/02 |
| Y | * Seite 3, Zeile 1 - Zeile 6; Ansprüche 1,3 * | 2,3 | B01J35/02 B01J35/10 |
| | * Seite 5, Zeile 16 - Seite 6, Zeile 7 * | | B01J37/08 |
| | * Seite 18, Zeile 1 - Zeile 14 * | | B01J37/00 |
| | * Seite 20, Zeile 6 - Seite 21, Zeile 11; Beispiel 3 * | | C07C29/17 C07C31/20 |
| | ----- | | B01J23/755 |
| X | WO 2012/101550 A1 (BASF SE [DE]; BASF CHINA CO LTD [CN]; BEERS ANNEMARIE [NL]) 2. August 2012 (2012-08-02) | 1-17 | B01J35/04 |
| | * Seite 6; Beispiel 1 * | | ADD. |
| | * Seite 5, letzter Absatz - Seite 6, Absatz 1 * | | B01J37/02 |
| | ----- | | |
| Y | US 7 612 241 B1 (WHITE DANIEL F [US] ET AL) 3. November 2009 (2009-11-03) | 2,3 | |
| | * Spalte 3, Zeile 65 - Spalte 4, Zeile 15 * | | |
| | ----- | | RECHERCHIERTE SACHGEBIETE (IPC) |
| T | ABDULLAH E C ET AL: "The use of bulk density measurements as flowability indicators", POWDER TECHNOLOGY, ELSEVIER SEQUOIA, LAUSANNE, CH, Bd. 102, Nr. 2, 3. Mai 1999 (1999-05-03), Seiten 151-165, XP002272427, ISSN: 0032-5910, DOI: 10.1016/S0032-5910(98)00208-3 * Seite 151, linke Spalte, Absatz 1. * | 1,12 | B01J C07C |
| | ----- | | |

Der vorliegende Recherchenbericht wurde für alle Patentansprüche erstellt

| Recherchenort | Abschlußdatum der Recherche | Prüfer |
|---|---|---|
| München | 31. März 2017 | Beckmann, Oliver |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 19 1735

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-03-2017

| Im Recherchenbericht angeführtes Patentdokument | Datum der Veröffentlichung | Mitglied(er) der Patentfamilie | Datum der Veröffentlichung |
|---|---|---|---|
| WO 02055453 A2 | 18-07-2002 | CN 1486291 A<br>DE 10101646 A1<br>EP 1351900 A2<br>JP 2004517135 A<br>US 2002193618 A1<br>WO 02055453 A2 | 31-03-2004<br>18-07-2002<br>15-10-2003<br>10-06-2004<br>19-12-2002<br>18-07-2002 |
| WO 2012101550 A1 | 02-08-2012 | CA 2823676 A1<br>CN 103339093 A<br>EP 2668148 A1<br>JP 2014507270 A<br>KR 20140004733 A<br>WO 2012101550 A1 | 02-08-2012<br>02-10-2013<br>04-12-2013<br>27-03-2014<br>13-01-2014<br>02-08-2012 |
| US 7612241 B1 | 03-11-2009 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 1628190 A
- US 1915473 A
- US 2139602 A
- US 2977327 A
- US 2967893 A
- EP 0340970 A2
- DE 2004611 A1
- DE 2004611 A
- EP 0807464 A1
- EP 1833778 B1

**In der Beschreibung aufgeführte Nicht-Patentliteratur**

- Butanediols, Butenediol, and Butynediol. **BEISPIEL.** Ullmann's Encyclopedia of Industrial Chemistry. 15. Juni 2000
- *J. Am. Chem. Soc.,* 1938, vol. 60, 309-319
- The Guide 2000 of Technical Foams. 33-41